# DEMANDE DE BREVET EUROPEEN

(11) **EP 0 951 865 A1**
(43) Date de publication de la demande: **27.10.1999**
(21) Numéro de dépôt: 99400959.5
(22) Date de dépôt: 20.04.1999
(51) Int. Cl.: A61B 5/107, A43D 1/02

(54) **Système de relevé d'une forme tridimensionnel, notamment d'une voûte plantaire, et procédé de realisation d'une chaussure ou d'une semelle orthopédique mettant en oeuvre le système**

(30) Priorité: 21.04.1998 FR 9804981
(71) Demandeur: KREON INDUSTRIE, 87000 Limoges (FR)
(72) Inventeur: Rebiere, Christian, 31460 Caraman (FR)
(74) Mandataire: Thibon-Littaye, Annick

(57) **Abrégé**

L'invention concerne un système (7) de relevé d'une forme d'un objet tridimensionnel (2), notamment d'une voûte plantaire. Il comprend un dispositif palpeur (1) à aiguilles (3). La voûte plantaire repose sur les extrémités supérieures (300) des aiguilles (3), de manière à les repousser vers le bas. L'ensemble des extrémités inférieures (301) définit une surface (*S*_{*A*}) répliquant la forme de voûte plantaire. On associe des ressorts tarés aux aiguilles (3) pour maîtriser leur enfoncement. Un dispositif (5) à capteur optique (50) génère un faisceau laser lamellaire (*F*_{*l*}) balayant les extrémités inférieures (301). Deux caméras (52, 53) analysent la trace du faisceau (*F*_{*l*}) et délivrent des signaux de sortie (*V*_{*S*}) représentant les coordonnées des secondes extrémités (301) de tiges (3). L'ensemble est couplé à un calculateur à programme enregistré (8) et à un système d'exploitation des données acquises à commande numérique. Dans d'autres modes de réalisation, l'acquisition de forme est réalisée, soit par un procédé photographique mettant en oeuvre un appareil numérique, soit par un procédé télémétrique, optique ou magnétique.

## Description

L'invention concerne un système de relevé d'une forme tridimensionnelle. Elle concerne plus particulièrement, bien que non exclusivement, un système de relevé de la forme d'une voûte plantaire. Un tel système est utilisé, par exemple, pour la réalisation de chaussures sur mesure.

De façon plus particulière, elle a pour objet un procédé de réalisation de chaussures ou de semelles orthopédiques faisant application du système. Pour fixer les idées, et sans que cela soit limitatif en quoi que ce soit de la portée de l'invention, on se placera dans ce qui suit dans ce cadre d'application préféré.

Pour une application de ce type, il est nécessaire de connaître la forme de la voûte plantaire de l'un ou des deux pieds d'un usager ou patient, en vue de réaliser des chaussures orthopédiques adaptées, ou pour le moins d'accessoires pour de telles chaussures, telles des semelles, par exemple.

De nombreux procédés ou dispositifs sont mis en oeuvre dans l'art connu, que l'on peut diviser arbitrairement en deux grandes catégories.

Une première catégorie concerne des dispositifs ou procédés que l'on peut qualifier de "analogiques". A titre d'exemple, on peut procéder par moulage de la forme du pied. L'usager pose son pied sur un bloc de matériau malléable, à base de silicones par exemple. Du fait de la pression exercée, le bloc de matériau conserve l'empreinte du pied et peut servir de matrice pour la réalisation ultérieure d'une chaussure ou d'une semelle orthopédique.

La précision obtenue par cette méthode n'est pas très élevée. En outre, le passage de la matrice ainsi acquise au "produit fini", c'est-à-dire la chaussure ou la semelle n'est pas aisé, ni rapide. Le procédé nécessite de nombreuses étapes manuelles.

En outre, il est généralement nécessaire d'obtenir, non pas une seule empreinte, mais plusieurs pour simuler au mieux divers états physiques : pied au repos (à plat), pied reposant sur la pointe et pied reposant sur le talon, par exemple, ce qui rend plus complexe le processus.

La seconde catégorie concerne des dispositifs et procédés de type numérique.

On connaît notamment de nombreux systèmes à capteur optique, notamment celui divulgué par le brevet français N° FR-B-2 685 764, au nom de la Demanderesse, intitulé "Capteur optique compact et à haute résolution pour l'analyse de formes tridimensionnelles". Ce système permet l'acquisition de profils de pièces tridimensionnelles, à surface gauche, et leur numérisation. Pour ce faire, le système comporte un capteur optique muni d'une source de rayonnement laser et une ou plusieurs caméras analysant la trace formée par le faisceau laser sur l'objet à étudier.

Plus précisément, le système engendre un "plan" laser, c'est-à-dire un faisceau lamellaire sectoral de très faible épaisseur, mais dont la largeur lui permet de couvrir tout ou partie de l'objet à analyser, avec les caméras précitées observant la trace du faisceau sous des incidences différentes. L'acquisition et la numérisation s'effectuent par balayage de la surface de l'objet par le plan de lumière laser.

Dans le cas des applications visées par l'invention, un tel système à capteur optique n'est pas utilisable tel quel pour l'acquisition et la numérisation du profil de la voûte d'un pied, essentiellement du fait que la surface dont on doit relever le profil se trouve sous le pied et qu'elle est inaccessible dans la mesure où l'on cherche à l'examiner quand le patient est en station debout ou en position de marche.

Ceci interdit de tirer plein profit des avantages que les capteurs optiques présentent par ailleurs, en permettant notamment d'atteindre une grande résolution dans la définition du profil tridimensionnel par une image de points dont les coordonnées sont connues par rapport à un référentiel (usuellement un plan horizontal) et d'assurer une acquisition rapide de grande précision ainsi que la numérisation de la forme acquise, ce qui permet une exploitation immédiate ou différée des mesures effectuées. En effet, les données acquises peuvent être traitées par un calculateur à programme enregistré, pour être délivrées à un dispositif les exploitant (machine outil à commande numérique par exemple), visualisées sur un écran, et/ou stockées dans une mémoire de masse pour un usage ultérieur, *in situ* ou à distance.

L'invention se fixe donc pour but de pallier les défauts des dispositifs de l'art connu et de répondre aux besoins, tout en conservant les avantages propres à un système à base d'un capteur optique, notamment celui d'opérer sans contact. Elle permet aussi de satisfaire à d'autres exigences qui sont associées à un système utilisé pour les applications visées préférentiellement par l'invention (simplicité, manipulation facile, transport aisé de l'appareil) et sans qu'il soit besoin de poser le pied sur une paroi transparente qui perturberait les mesures en introduisant des déviations du faisceau optique de détection.

Pour ce faire, le système de relevé de forme d'un objet tridimensionnel selon l'invention comprend un dispositif palpeur, ayant la forme d'un cadre ou d'une boîte, et muni d'une matrice d'aiguilles élastiquement rétractables, sur le dessus desquelles repose la voûte plantaire. Du fait de la force verticale exercée (due au poids de l'usager), les aiguilles s'enfoncent, par translation sur elles-mêmes de haut en bas. Les aiguilles sont associées à des moyens de rappel élastique, tels que des ressorts, de manière à maîtriser cet enfoncement.

L'ensemble des extrémités inférieures reproduit le profil exact de la voûte du pied ou de la partie de voûte reposant sur les aiguilles. De façon plus générale, si un objet tridimensionnel quelconque exerce une force de pression sur des premières extrémités des aiguilles, le profil de la face d'appui est reproduit par l'ensemble des secondes extrémités des aiguilles. On admet ici, bien que cela ne soit pas obligatoire, que toutes les aiguilles ont même longueur.

Le système selon l'invention comprend enfin un dispositif d'acquisition de forme disposé sous le dispositif palpeur à aiguilles, de manière à acquérir un nuage de points représentatif du profil de la surface formée par les extrémités inférieures des aiguilles et à en assurer la numérisation.

Selon un premier mode de réalisation, le dispositif d'acquisition de forme et de numérisation est constitué par un capteur optique mettant en oeuvre au moins une caméra opto-électronique et au moins une source laser. Le relevé s'effectue par balayage de la surface enveloppe des extrémités inférieures des aiguilles à l'aide d'au moins un faisceau laser. Les signaux de sortie générés par le capteur optique, représentant des données de coordonnées des points du nuage, sont ensuite transmis à un organe de traitement de signal, après une numérisation préalable éventuelle. Les données acquises peuvent être exploitées directement par tout dispositif approprié (machine-outil à commande numérique, etc.) et/ou stockées pour un usage ultérieur.

Lorsque la force d'appui est relâchée, les aiguilles reprennent leur position de repos, car elles sont repoussées par les ressorts tarés. Dans cette position dite de "repos" (avant et après acquisition), l'ensemble des extrémités inférieures des aiguilles forme une surface pouvant servir de référence pour un calibrage initial du dispositif à capteur optique. En général, cette surface est plane, et elle se dispose horizontalement le plus souvent.

Selon un deuxième mode de réalisation, on met en oeuvre un dispositif d'imagerie. L'acquisition de forme est effectuée à l'aide d'un appareil photographique ou d'une caméra vidéo numérique. Les extrémités inférieures des aiguilles sont éclairées par une source de lumière. L'amplitude de l'enfoncement peut être déterminé par le biais des variations de densité lumineuse. Les signaux électroniques de sortie de l'appareil photographique ou de la caméra sont transmis à une carte d'acquisition numérique d'un micro-ordinateur ou d'un appareil similaire, et sont traités à l'aide d'un logiciel de traitement d'image.

Selon un troisième mode de réalisation, on met en oeuvre un dispositif de télémétrie optique ou magnétique, dispositif lui-même susceptible de plusieurs variantes. Le dispositif de télémétrie, qui peut être constitué à base d'un réseau de diodes laser semi-conductrices, est placé sous la matrice d'aiguilles. Le dispositif de télémétrie mesure la distance le séparant de chacune des extrémités inférieures des aiguilles, et donc peut déterminer leurs enfoncements par rapport à un plan de référence.

Dans une variante préférée du système selon l'invention, le dispositif palpeur est équipé de moyens de rappel élastiques résistant à l'enfoncement des aiguilles, qui sont constituées de manière à permettre un réglage de la force de résistance exercée, afin de tenir compte notamment du poids et de la morphologie de l'usager. On peut notamment prévoir trois positions prédéfinies pour une plaque permettant ainsi de modifier le tarage de ressorts individuellement associés aux différentes aiguilles et comprimés entre cette plaque et une paroi fixe, toutes deux traversées par les aiguilles.

L'invention sera mieux comprise et d'autres caractéristiques et avantages apparaîtront à la lecture de la description qui suit en référence aux figures annexées, parmi lesquelles :
- les figures 1A et 1B illustrent, en coupe et vue de côté, un exemple de réalisation d'un dispositif palpeur à aiguilles et ressorts tarés, constituant l'un des composants du système de relevé de la voûte d'un pied selon l'invention, au repos et en cours de mesures d'acquisition de forme, respectivement ;
- la figure 2 illustre ce même dispositif en vue de dessus ;
- la figure 3 illustre une variante du dispositif palpeur des figures 1A à 2 ;
- la figure 4 illustre un système complet de relevé de forme d'objet tridimensionnel, selon un premier mode de réalsiation, incluant un dispositif à capteur optique ;
- la figure 5 illustre un exemple de réalisation pratique du système de la figure 4 ;
- les figures 6A à 6B illustrent un dispositif d'acquisition et de numérisation selon un mode de réalisation supplémentaire de l'invention;
- les figures 7A à 7B illustrent un dispositif d'acquisition et de numérisation selon un autre mode de réalisation supplémentaire, décrit sous deux variantes.

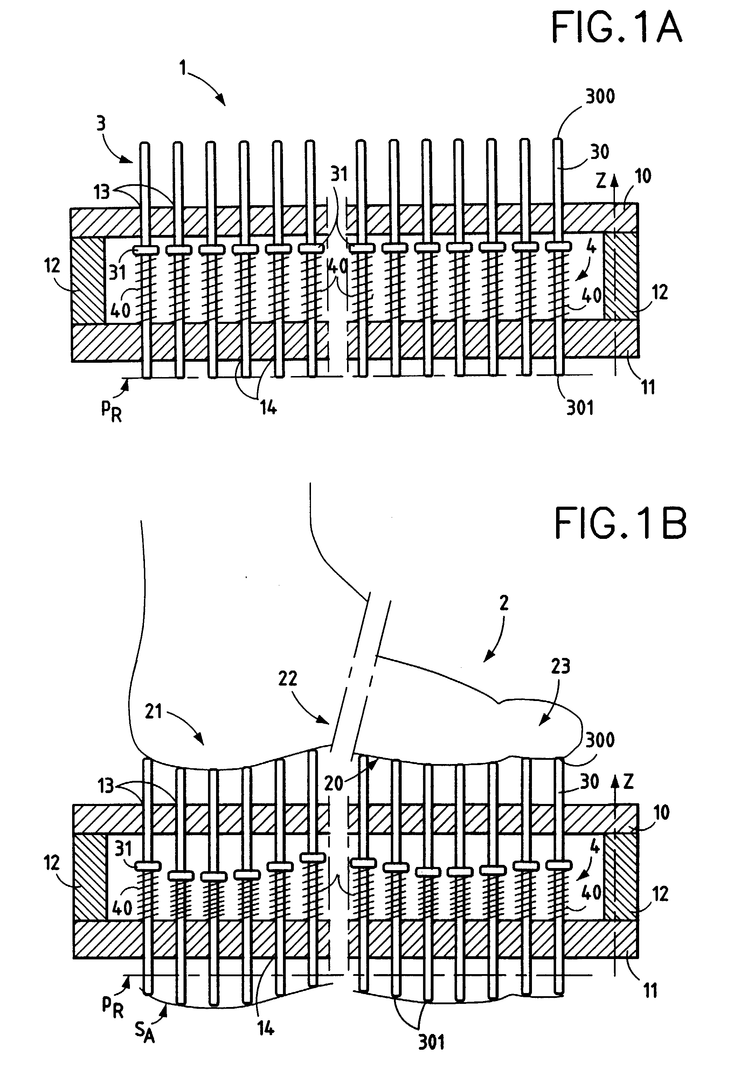

On va maintenant décrire un exemple de réalisation d'un système d'acquisition et de numérisation de la surface de la voûte d'un pied selon l'invention, et tout d'abord un exemple de réalisation de l'un de ses composants essentiels, le dispositif palpeur 1 par référence aux figures 1A à 2.

Le dispositif palpeur 1 est constitué d'une boîte à aiguilles rétractables 1. La structure de la boîte à aiguilles 1 comprend essentiellement des parois, que l'on appellera arbitrairement supérieure 10 et inférieure 11, ou parois principales parallèles, maintenues à une distance constante l'une de l'autre par des parois latérales ou des entretoises 12. Les parois supérieure 10 et inférieure 11 sont percées d'orifices, 13 et 14, respectivement, et sont avantageusement substantiellement planes. Ces orifices, 13 et 14, laissent le libre passage aux corps cylindriques 30 d'un jeu d'aiguilles ou tiges 3, de sorte que ces corps cylindriques 30 peuvent être animés d'un mouvement de translation suivant un axe Z orthogonal aux parois 10 et 11, *a priori* un axe vertical dans l'application préférée. Les extrémités supérieures 300 et inférieures 301 des corps cylindriques 30 dépassent des parois supérieure 10 et inférieure 11, respectivement.

Les corps cylindriques 30 sont munis de butées 31 proches de la paroi supérieure 10. Un jeu 4 de ressorts 40, disposés comprimés entre la paroi inférieure 10 et ces butées 31, repousse les aiguilles 3 vers le haut (dans l'exemple de la figure 1A). Les ressorts 40 sont coaxiaux aux corps cylindriques 30. Dans un état que l'on appellera de "repos", illustré par la figure 1A, c'est-à-dire lorsque aucune force d'appui n'est exercée sur les extrémités supérieures 300 des aiguilles 3, celles-ci sont en position que l'on appellera "haute". L'ensemble des extrémités inférieures 301 de ces aiguilles 3 forme une surface substantiellement plane qui définit un plan de référence *p*_{*R*}*.* On peut d'ailleurs faire en sorte que les butées 31 viennent en contact avec la face intérieure de la paroi supérieure 10, de manière à ce que les positions de repos des aiguilles soient bien définies. Si cette face présente une bonne planéité, le plan de référence *P*_{*R*} est lui aussi défini avec une bonne précision.

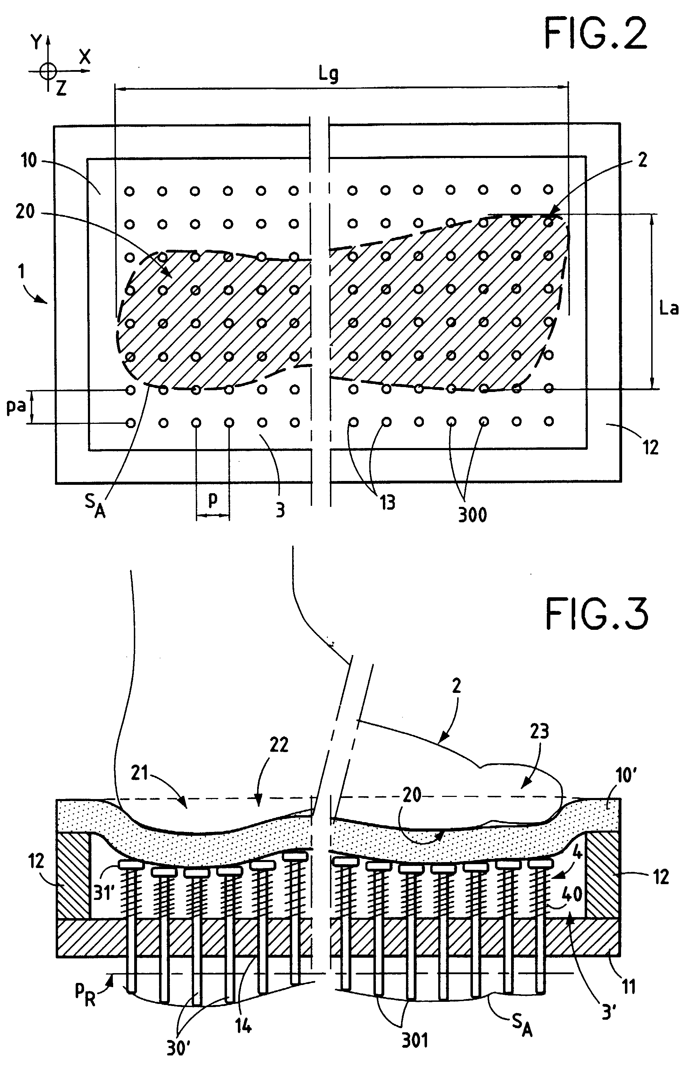

De façon préférentielle, comme illustré plus particulièrement par la figure 2, montrant le dispositif 1 en vue de dessus, les aiguilles 3 sont distribuées régulièrement aux intersections des colonnes et des rangées d'une matrice rectangulaire, c'est-à-dire suivant deux axes orthogonaux *X* et *Y*, eux-mêmes orthogonaux à l'axe *Z* précité.

Lorsque l'usager (non représenté) pose son pied 2 sur les aiguilles 3, de façon plus précise sur leurs extrémités supérieures 300, celles-ci, sous la pression exercée, s'enfoncent. Selon une caractéristique importante de l'invention, la translation des tiges sur elles-mêmes s'effectue à l'encontre des ressorts tarés 40. Ceux-ci, étant emprisonnés entre la paroi inférieure 11 et les butées 31, sont plus ou moins comprimés, en fonction de l'amplitude des forces d'appui localement exercées sur telle ou telle aiguille 3. Le tarage peut être obtenu, soit intrinsèquement en utilisant des ressorts d'une raideur prédéterminée, soit en exerçant une précontrainte, les ressorts étant déjà comprimés à l'état de repos (figure 1A) entre les butées 31 et la paroi inférieure 11.

A titre d'exemple, comme illustré par la figure 1B, le pied 2 étant supposé reposant à plat sur l'ensemble des aiguilles 3, le talon 21 (partie gauche de la figure 1B) exerce une force de compression plus élevée que la pointe du pied 23 (partie droite). La partie centrale 22 de la voûte plantaire 20, en retrait vers le haut exerce une force de pression encore moins élevée. Il s'ensuit que les extrémités inférieures 301 des aiguilles 3 dépassent plus ou moins de la paroi inférieure 11. L'ensemble de ces extrémités 301 d'aiguilles 3 définit, par interpolation, une surface *S*_{*A*} qui reproduit le dessous du pied 2, c'est-à-dire le profil de la voûte plantaire 20 que l'on désire acquérir.

On doit remarquer que, au moins à l'instant initial où le pied 2 est posé sur le dispositif 1, tous les ressorts 40 opposant une résistance identique à la force d'appui, la reproduction précitée est fidèle, car le profil de la surface d'appui (extrémités supérieures 300 des aiguilles 3) est déterminé par le profil de la voûte plantaire 20 et non l'inverse, comme ce serait le cas si le profil de la surface d'appui était préétabli. Les dispositions spécifiques à l'invention permettent donc d'obvier aux inconvénients présentés par les systèmes à capteurs optiques de l'art connu et précédemment signalés.

Pour que l'acquisition de forme s'effectue avec une bonne précision, il est nécessaire que le nombre d'aiguilles 3 soit suffisant. En outre, un nombre insuffisant d'aiguilles fausserait la mesure pour une autre raison : la matière vivante du pied étant malléable, l'enfoncement superficiel des différentes aiguilles 3 serait différent d'un endroit à l'autre de la voûte plantaire 20, selon la pression locale exercée. Ce facteur d'erreur est minimisé lorsque la densité d'aiguilles 3 augmente. Enfin, il est clair que le processus d'acquisition de forme ne doit pas causer des désagréments à l'usager, voire des douleurs, ce qui pourrait être le cas si le nombre d'aiguilles 3 était insuffisant.

Pour que le dispositif 1 convienne à différents usagers, il est en outre souhaitable que le réglage de la force de répulsion des ressorts 40, c'est-à-dire leur tarage, soit adapté au poids et à la morphologie de l'usager. Pour ce faire, deux méthodes principales peuvent être utilisées.

Selon une première méthode, on utilise une gamme de dispositifs interchangeables. A titre d'exemple pratique, on peut concevoir trois modèles distincts de dispositifs distincts : un premier modèle étant destiné à des adultes, un second à des enfants ou des adolescents, et un troisième à des enfants en bas âge, voire à des bébés. Les dimensions du dispositif 1, le nombre des aiguilles 3 et, surtout, le tarage individuel des ressorts 40 varient d'un modèle à l'autre.

Selon une seconde méthode, on utilise un modèle de boîte à aiguilles unique quels que soient l'âge et le poids du patient. Dans ce cas, il est utile de pouvoir conserver une bonne amplitude de déplacement des tiges, ce pour quoi on prévoit de régler la force de rappel élastique des ressorts, c'est-à-dire le tarage des ressorts. On peut, par exemple, agir sur leur précontrainte. Celle-ci peut être ajustée en rapprochant ou en éloignant les parois supérieure 10 et inférieure 12 l'une de l'autre, à l'aide d'un système à vis réglable, remplaçant en tout ou partie les parois latérales 12. Les ressorts restent disposés entre les butées 31 et la paroi inférieure 11. De la sorte, on modifie simultanément le tarage pour toutes les tiges.

On peut aussi, et de manière préférentielle, équiper le boîtier 1 d'une plaque intermédiaire montée coulissante sur les parois latérales 12 ou sur des tirants équivalents pour définir le plan d'appui des butées 31. Il suffit alors de régler la position de cette plaque en hauteur pour obtenir le tarage désiré. Le cas échéant, on peut prévoir des tarages prédéterminés correspondant par exemple à un adulte relativement lourd, à un adulte de faible poids ou un adolescent, à un enfant. On peut imposer à la plaque de réglage la position de trois niveaux ainsi désirés, au moyen par exemple d'un système de came à commande manuelle agissant simultanément aux quatre angles de la boîte.

On va maintenant décrire, par référence à la figure 3, une variante de réalisation du dispositif à aiguilles, référencé 1'. Les éléments identiques aux figures précédentes portent les mêmes références et ne seront redécrits qu'en tant que de besoin.

Selon cette variante de réalisation, la paroi supérieure, référencée 10', n'est plus rigide mais réalisée en matériau à propriétés élastiques ou pour le moins réver-siblement déformable sous l'effet d'une force de pression. Cette paroi 10' est pleine, c'est-à-dire non munie d'orifices comme précédemment. Les aiguilles, ici référencées 3' ont subi également des modifications : la butée, référencée 31', fait également office d'extrémité supérieure du corps cylindrique 30' et est en appui sur la face inférieure de la paroi 10'. Par contre, les aiguilles 3' sont enfilées, comme précédemment, dans les orifices 14 de la paroi inférieure 11, cette dernière faisant office de plaque de guidage les maintenant verticales. De même, subsistent les parois latérales 12, sur les extrémités supérieures desquelles est fixée la paroi déformable 10'.

Lorsque l'usager pose un pied 2 sur la paroi 10', celle-ci s'enfonce et, par appui sur les butées 31' elle entraîne les corps cylindriques 30' des aiguilles 3'. Les ressorts associés à ces aiguilles 3' exerce une force de résistance qui tend à s'opposer à l'enfoncement de la paroi 10' et au mouvement de translation des aiguilles 3' vers le bas. Comme précédemment également, les ressorts 40 sont tarés. De ce fait, la paroi 10' épouse le profil de la voûte plantaire 20 du pied et l'ensemble des extrémités inférieures 301 des aiguilles 3' définissent une surface gauche *S*_{*A*} reproduisant, sous le dispositif 1', le profil précité.

L'acquisition de ce profil s'effectue par la mesure des déplacements des extrémités 301 des aiguilles 3' par rapport à un plan de référence *p*_{*R*} (position de repos lorsque le pied 2 n'est pas posé sur la paroi 10'). La position initiale de la paroi 10', ou position de repos, est représentée sur la figure 3 en traits pointillés. Cette position est également reprise par la paroi 10' lorsque la pression est relâchée (retrait du pied 2), d'une part sous l'effet des ressorts 40, d'autre part, si la paroi 10' est en matériau élastique, du fait de ces propriétés élastiques intrinsèques.

Bien entendu, les butées 31' pourraient aussi être éloignées de la tête supérieure des tiges et reposer sur une plaque intermédiaire de réglage du tarage comme ci-dessus.

Dans les deux variantes de réalisation, les dispositifs, 1 ou 1', "déportent" le profil de la voûte 20 dans un lieu de l'espace (surface *S*_{*A*}) où il devient plus accessible et peut être acquis et numérisé de façon aisée. Autrement dit, la surface définie par les extrémités inférieures des tiges constitue une réplique du profil imposé à leurs extrémités supérieures. Il est important de noter toutefois que la reproduction du profil à l'identique suppose que toutes les tiges ont la même longueur, mais que ceci n'est pas obligatoire, car le profil peut également être calculé à partir du déplacement en translation des différentes tiges par rapport à un profil de référence qui n'est pas nécessairement plan.

Conformément à l'invention, le dispositif palpeur tel que décrit est particulièrement adapté pour être associé à une détection de la position des tiges (extrémités inférieures) s'effectuant par des moyens optiques qui observent leurs faces terminales, ce qui serait impossible en opérant sur leurs extrémités supérieures.

Dans un premier mode de réalisation qui va maintenant être décrit, on met en oeuvre un dispositif à capteurs optiques. Il doit être noté que, grâce aux dispositions adoptées dans le cadre de l'invention, l'acquisition de forme peut s'effectuer directement, c'està-dire sans que le rayonnement laser doive traverser une paroi transparente intermédiaire, formant support de l'objet dont le profil est à acquérir, en l'occurrence du profil de la voûte d'un pied dans l'application préférée.

Le profil de la surface *S*_{*A*} est acquis par un dispositif à capteur optique disposé sous le dispositif palpeur à aiguilles 1. Il doit être bien entendu que la surface *S*_{*A*} est bornée. Comme le montre plus particulièrement la figure 2, elle est délimitée dans la pratique par les dimensions de la voûte 20 du pied 2, c'est-à-dire la zone d'appui sur les extrémités 300 des aiguilles 3 : aire hachurée en pointillés. En dehors de cette aire d'appui, les aiguilles 3, et plus précisément leurs extrémités supérieures 300, ne sont pas sollicitées. Sous le dispositif palpeur 1, on retrouve cette caractéristique : une zone centrale délimitant l'aire de surface *S*_{*A*} (courbe gauche en trois dimensions) et une zone périphérique plane confondue avec le plan de référence *P*_{*R*}*,* représentant l'état de repos.

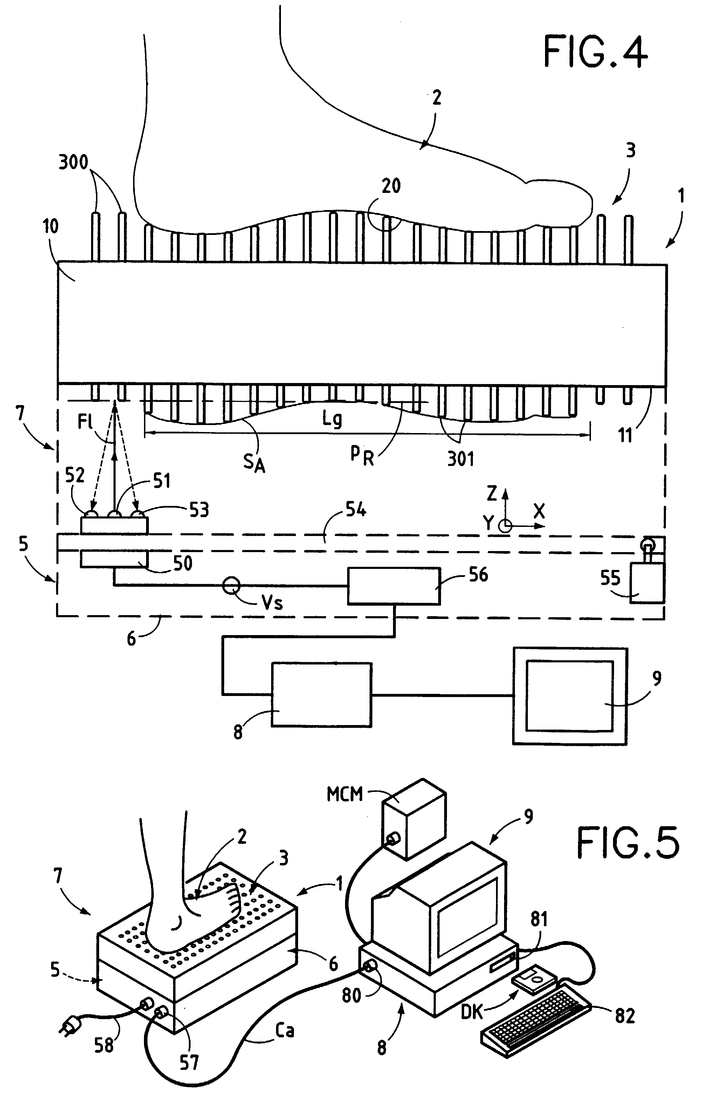

La figure 4 illustre un exemple de réalisation du système complet 7, conforme au premier mode de réalisation, incorporant un dispositif à capteur optique.

Dans un but de simplification, on n'a représenté que les éléments essentiels à la bonne compréhension de l'invention. On s'est placé dans le cas de la variante des figues 1A à 2, sans que cela soit limitatif de la portée de l'invention. Les éléments communs aux figures précédentes portent les mêmes références et ne seront redécrits qu'en tant que de besoin.

Le système 7 comprend un dispositif palpeur 1 à aiguilles 3 et un dispositif à capteur optique 5, le tout étant supporté par un boîtier unique ou bâti 6 (en traits pointillés sur la figure 4).

Le dispositif 5 est susceptible de plusieurs variantes de réalisation. La figure 4 illustre une première variante de réalisation pour laquelle le dispositif 5 comprend un capteur optique 50 se déplaçant linéairement parallèlement à l'un des axes horizontaux de la matrice d'aiguilles 3 du dispositif de palpation 1, avantageusement suivant l'axe *X* qui est supposé parallèle à la plus grande longueur du boîtier ou du cadre emprisonnant les aiguilles 3.

Pour ce faire, on prévoit un moteur d'entraînement 55, solidaire du bâti 6, et un jeu de poulies et/ou d'engrenages classiques (non représenté). Il peut s'agir, par exemple, d'une vis sans fin entraînée en rotation par le moteur 55 et entraînant, à son tour, le capteur 50 dans un mouvement de translation parallèlement à l'axe *X*. On peut encore, puisqu'il s'agit de mesures discrètes, comme il le sera montré ci-après, utiliser un moteur 55 du type pas-à-pas. Le capteur 50 est disposé sur un équipage mobile et guidé, dans son mouvement de translation suivant l'axe *X*, par des rails 54, disposées de part et d'autre de ce capteur, parallèlement à cet axe *X* et solidaire du bâti 6. De façon duale, dans une variante non représentée, le capteur 50 pourrait être muni d'un moteur couplé à une crémaillère ou une vis sans fin, par exemple, solidaire des rails de guidage 54 ou du bâti 6. On peut également mettre en oeuvre des transducteurs classiques (non représentés) permettant de connaître, à tout instant, la position exacte du moteur 55 le long de l'axe *X*, associés éventuellement à des détecteurs de fin de course.

Le capteur 50 peut être semblable, voire identique, au capteur décrit dans le brevet français FR-B-2 685 764 précité, auquel on se reportera avec profit pour une description plus détaillée. Le capteur 50 comprend essentiellement une source laser 51 générant un faisceau lamellaire *F*_{*1*}*.* La source 51 est avantageusement constituée d'une diode laser semi-conductrice. Le capteur 50 est muni d'au moins une caméra et préférentiellement de deux caméras, 52 et 53, comme illustré par la figure 4, disposées de part et d'autre de la source laser 51, suivant l'axe de déplacement du capteur 50, c'est-à-dire suivant l'axe *X* (dans l'exemple décrit).

Le faisceau lamellaire *F*_{*1*} est dirigé vers les extrémités inférieures 301 des aiguilles 3. De façon préférentielle, il frappe le plan de référence *P*_{*R*} sous une incidence normale. De façon avantageuse, la distance séparant le capteur 50 du plan de référence *P*_{*R*} et l'angle au sommet du faisceau *F*_{*1*} sont choisis de telle sorte que, quelle que soit l'enfoncement des aiguilles 3 (position extrême possible des extrémités 301 des aiguilles 3), le faisceau incident, qui se traduit par un trait de lumière étroit, couvre toute la largeur de la paroi inférieure 11 (suivant l'axe *Y*, dans l'exemple décrit). Mais bien entendu, en variante on peut aussi prévoir un balayage en deux directions.

Les caméras 52 et 53 sont inclinées par rapport à l'axe Z, orthogonal au plan *P*_{*R*}*,* de manière à analyser la trace du faisceau lamellaire *F*_{*1*} sur les extrémités 301 des aiguilles 3, suivant des angles différents. Ces caméras délivrent des signaux électriques de sortie *V*_{*S*} qui sont transmis à un ensemble de traitement de signaux 8. Ce dernier peut être situé dans le boîtier 7 ou, comme suggéré sur la figure 4, à l'extérieur de celui-ci. Dans ce dernier cas, il peut s'agir avantageusement d'un calculateur numérique à programme enregistré classique, par exemple un micro-ordinateur 8 connecté à un organe de visualisation 9, par exemple un écran cathodique ou un écran à cristaux liquides ou à plasma. Si les caméras 52 et 53 sont du type analogique, les signaux *V*_{*S*} doivent tout d'abord être convertis en signaux numériques. Pour ce faire, il est fait usage d'un convertisseur analogique-numérique classique (non représenté) disposé à l'intérieur du châssis 6 ou d'une carte spécialisée d'acquisition de signaux analogiques située à l'intérieur de l'ensemble de traitement de signaux 8. Il existe également des caméras qui délivrent directement des signaux numériques représentatifs de l'image captée. Dans ce cas, les signaux sont transmis à une carte disposée dans l'ensemble 8 et munie d'un port d'acquisition de signaux numériques. Le boîtier 7 peut comprendre également des circuits de prétraitement 56 convertissant les signaux de sortie *V*_{*S*} en des signaux conformes à un protocole normalisé, avant de les transmettre sous cette forme à l'ensemble de traitement de signaux, par exemple à un port série "RS 232" standard.

L'acquisition du profil de la voûte 20 d'un pied 2, via le profil de la surface *S*_{*A*} qui en est l'image, s'effectue classiquement par tranches successives que l'on appellera *S*_{*i*}, *i* étant un indice arbitraire variant par exemple entre 1 et une valeur maximale *i*ₘₐₓ. Dans le cadre de l'invention, chaque tranche S_{*i*} représente préférentiellement une colonne (suivant l'axe *Y*) de la matrice rectangulaire d'extrémités 301 d'aiguilles 3. Plus précisément, dans l'exemple décrit, une tranche S_{*i*} représente la position de toutes les extrémités 301 des aiguilles 3 d'une colonne d'indice *i* de la matrice par rapport au plan de référence *P*_{*R*}*,* c'est-à-dire l'amplitude de l'enfoncement des aiguilles 3 correspondantes.

Si *N* et *M* sont les nombres respectifs d'aiguilles 3 par colonne (suivant l'axe *Y*) et par ligne (suivant l'axe *X* ou axe de déplacement du capteur 50), le nombre de points acquis par tranche S_{*i*} est, au maximum *N*, et le nombre de tranches maximum est égal à *i*_{*max*} = *M.* Dans la généralité des cas, le nombre de tranches utiles est inférieur : il dépend de la longueur *Lg* du pied 2, *Lg* étant entendu comme la longueur maximale de la voûte 20 en contact avec les extrémités supérieures 300 des aiguilles 3, compte tenu du pas *pg* entre ces extrémités (voir figure 2) suivant cette direction, puisqu'il s'agit d'une mesure discrète. De même, le nombre de points utiles, pour une tranche donnée S_{*i*}, dépend de la largeur de la voûte 20. Le nombre de points utiles maximum est déterminé par la largeur maximale *La* (voir figure 2) de la voûte plantaire 20 en contact avec les extrémités supérieures 300 des aiguilles 3, compte tenu du pas *pa* entre ces extrémités (voir figure 2) suivant cette direction.

On va maintenant décrire de façon plus détaillée le processus d'acquisition du profil de la voûte plantaire 20, ou plus généralement d'un objet tridimensionnel exerçant une force d'appui sur les extrémités supérieures 300 des aiguilles 3.

Le processus comprend une phase préliminaire de calibrage. Elle est effectuée dans des conditions initiales dites de "repos", c'est-à-dire sans qu'aucune contrainte soit exercée sur les extrémités supérieures 300 des aiguilles 3. La surface *P*_{*R*}*,* dite de référence, définie par les extrémités inférieures 301 des aiguilles 3, est balayée en son entier : acquisition de *M* tranches S_{*i*} de *N* points chacune, *i* variant de 1 à *i*_{*max*}*.* Pour ce faire, le capteur 50 est entraîné le long de l'axe *X* de façon à illuminer, tranche par tranche S_{*i*}, toute la surface de référence *P*_{*R*}*.* Dans une variante, le capteur devant, *a priori,* effectuer un aller et retour pour revenir à la position de départ, on peut effectuer une double acquisition et calculer une moyenne des deux séries de mesure.

Aux imprécisions près, la surface de référence est sensiblement représentée par un plan *P*_{*R*}*,* que l'on appellera de référence, mais la condition de planéité n'est pas nécessaire, car la phase ultérieure d'acquisition du profil cherché comprend une étape de comparaison avec les coordonnées des points de référence acquises. La surface de référence *P*_{*R*} définit en effet un nuage de points de coordonnées de référence.

Cette phase préliminaire de calibrage peut être effectuée une fois pour toutes, s'il est possible d'admettre que le système présente une stabilité dans le temps suffisante, à des périodes de temps déterminées ou avant chaque acquisition. Dans une variante préférée de l'invention, le dispositif palpeur 1 est amovible et interchangeable, de manière à pouvoir utiliser le même dispositif à capteur optique 5, quel que soit l'usager (par exemple adulte, enfant ou bébé). Dans ce cas, il est au moins nécessaire d'effectuer une phase de calibrage après chaque remplacement. Il en est de même quand on conserve le même dispositif palpeur en réglant plutôt le tarage des ressorts.

Les données représentant les coordonnées de référence sont traduites par l'ensemble de traitement de signaux en mots binaires, stockés dans une mémoire vive dont il est muni ou dans une mémoire de masse (disque dur, etc.) pour un usage ultérieur, c'est-à-dire pour la phase d'acquisition proprement dite d'un profil.

Cette phase comprend les étapes suivantes :
a/ pose du pied 2 sur les extrémités supérieures 300 des aiguilles 3, dans une posture donnée (à plat, sur le talon, etc.), c'est-à-dire en appui total ou partiel ;
b/ balayage de la surface formée par les extrémités inférieures 301 des aiguilles 3 par le faisceau lamellaire *F*_{*1*}*,* le balayage étant obtenu par un mouvement de translation du capteur 50 le long de l'axe *X* (éventuellement une acquisition double : aller et retour) ;
c/ acquisition de *M* tranches S_{*i*} de *N* points chacune et génération de signaux de sortie *V*_{*S*} issus d'une conversion opto-électronique réalisée par les caméras 52 et 53, de manière à relever les coordonnées des points acquis dans un espace de mesure donné ;
d/ et transformation des données acquises et numérisées dans un référentiel absolu par comparaison avec les données de coordonnées de référence appartenant à l'espace de mesure, c'est-à-dire les coordonnées des extrémités inférieures 301 lorsqu'elles sont dans le plan de référence *P*_{*R*}*,* de manière à relever le profil de la voûte plantaire 20.

Ces étapes peuvent être complétées par une étape d'interpolation et de lissage des coordonnées des points ainsi acquis, de manière à obtenir un profil à variation continue.

Comme précédemment, les coordonnées acquises se traduisent par des mots binaires qui peuvent être stockés, notamment si les signaux acquis ne sont pas utilisés immédiatement mais destinés à un usage différé.

En réalité, comme il a été indiqué, seule l'acquisition de l'aire correspondant à la surface *S*_{*A*} est utile. Pour éliminer les mesures parasites dues à des imprécisions, géométriques ou autres, il suffit de retenir un seuil audessous duquel les mesures de coordonnées sont écartées. Dans la pratique, les comparaisons de l'étape d/ consistent à calculer, pour une aiguille 3 donnée, l'amplitude de l'enfoncement de celle-ci par rapport à sa position de repos (position de son extrémité 301 sur la surface de référence *P*_{*R*}*).* Si cette amplitude est inférieure au seuil précité, celle-ci est ignorée, ce qui évite notamment la prise en compte de points en dehors de l'aire *S*_{*A*}, c'est-àdire en définitive en dehors de la voûte 20. Cette valeur de seuil peut être prédéterminée ou calculée à partir d'imperfections constatées et mesurées pendant la phase de calibrage. A titre d'exemple, le seuil pourrait être déterminé en calculant la valeur d'erreur maximale qui permettrait d'assimiler la surface de référence *P*_{*R*} à un plan. La précision de mesure obtenue lors de l'acquisition est au plus égale à la valeur du seuil ainsi adoptée ou calculée.

Les acquisitions du profil de la voûte 20 pour d'autres postures du même pied 2 (sur les talons, sur la pointe de pieds, etc.) ou de l'autre pied de l'usager s'effectue aisément en répétant les étapes d'acquisition précédentes. Il n'est généralement pas nécessaire d'effectuer une phase de calibrage entre chaque série d'acquisition.

En sus du stockage possible des données acquises, celles-ci peuvent être affichées sur l'organe de visualisation 9, en temps quasi-réel, après la fin de l'étape b/ de balayage. Cet affichage peut s'effectuer, de façon connue *per se*, de toute manière convenable : affichage dit "filaire" par exemple ou, si l'étape d'interpolation et de lissage est réalisée, sous la forme d'une surface tridimensionnelle à variation continue, que l'on peut faire tourner dans l'espace, à l'aide de programmes d'affichage appropriés, disponibles dans le commerce.

Enfin, dans l'application préférée de l'invention, lors d'une phase finale, les données d'acquisition, disponibles sous forme numérique, peuvent être utilisée directement ou en différé, pour la commande d'une machine outil à commande numérique pour la fabrication de composants de chaussures orthopédiques, notamment de la semelle intégrée à ces chaussures, ou d'une semelle orthopédique à placer dans une chaussure conventionnelle. Cette phase peut être effectuée *in situ* ou, au contraire dans un lieu distant. Dans ce dernier cas, les données peuvent être transmises par voie télématique (réseau local, Internet, etc.) ou être enregistrée sur un médium intermédiaire (disquette, bande magnétique, etc.).

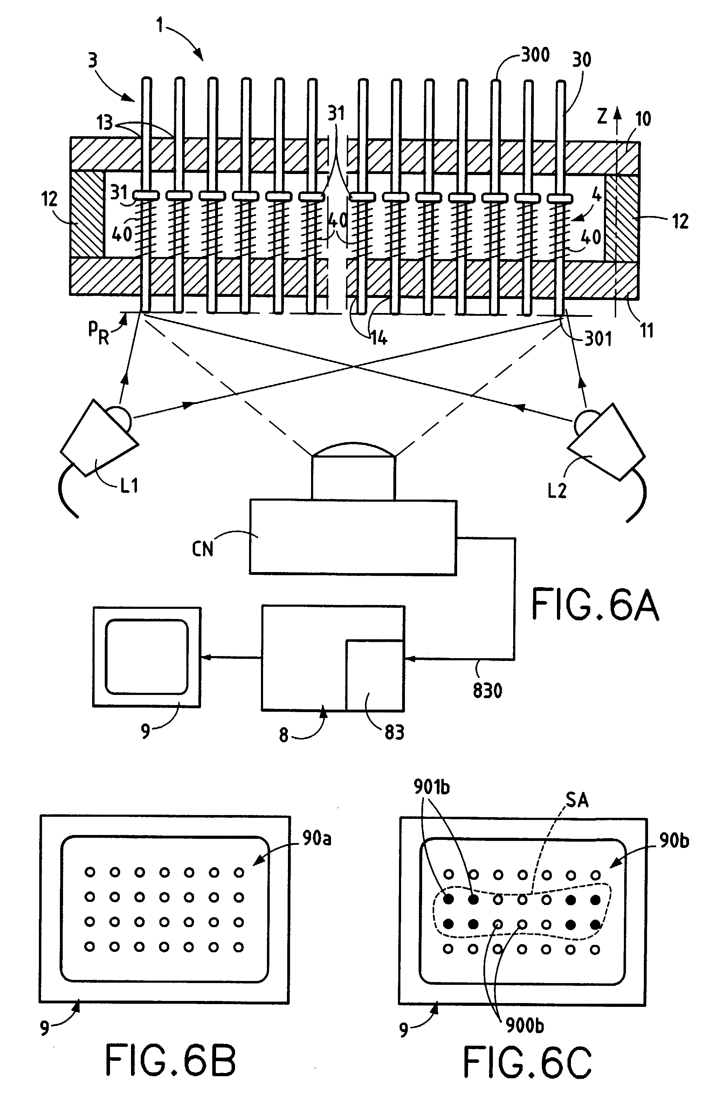

La figure 5 illustre un exemple de réalisation pratique du système de relevé de la figure 4.

Le système d'acquisition 7 comprend le dispositif 5 à capteur optique (figure 4 : 50) et le dispositif palpeur 1 à aiguilles 3 dans une même enceinte 6. De façon préférentielle, le dispositif palpeur 1 est amovible, comme il a été indiqué. De façon préférentielle également, l'alimentation en énergie électrique nécessaire aux caméras et à la source laser ainsi qu'à un éventuel circuit de prétraitement (figure 4 : 56) est autonome et amenée par un câble secteur 58. On prévoit aussi les différentes sources de tensions électriques nécessaires au bon fonctionnement de ces organes.

Dans l'exemple illustré sur la figure 5, le système de relevé proprement dit 7 est indépendant de l'ensemble de traitement de signaux, constitué par un micro-ordinateur 8, ce qui permet de le transporter aisément. La liaison avec le micro-ordinateur 8 est effectué par un simple câble *Ca,* par exemple une liaison de transmission bidirectionnelle de données séries, relié en une première extrémité à un connecteur 57 du boîtier 6, et en une seconde extrémité sur un connecteur 80 du micro-ordinateur 8. Le micro-ordinateur 8 est couplé à un organe de visualisation 9 et à un clavier d'entrée de données et/ou d'instructions 82. Il est muni des circuits classiques à ce type d'appareil : mémoire vive, unité centrale, mémoire de masse (disque dur), lecteur de cédérom, etc., non visibles sur la figure. Il comprend également un lecteur/enregistreur 81 de disquettes *DK,* sur lesquelles il est possible d'enregistrer tout ou partie des données acquises et numérisées. Les programmes, spécifiques ou non, nécessaires au bon fonctionnement du micro-ordinateur 8 sont chargés par l'intermédiaire de disquettes, d'un cédérom ou directement par télédéchargement à partir d'un réseau local ou externe (Internet, intranet, etc.). La numérisation peut s'effectuer, soit directement par des circuits présents dans le bâti 7 (figure 4 : 56), soit par une carte spécialisée du micro-ordinateur 8, recevant les données sous forme analogique par le câble *Ca*.

Le micro-ordinateur 8 effectue toutes les opérations de traitement de signaux nécessaire à l'acquisition de formes, mais aussi transmet des signaux de commande au capteur 50 (figure 4) et à ses circuits de motorisation, notamment pour effectuer les différents balayages nécessaires aux phases de calibrage et d'acquisition de coordonnées : déplacements aller et retour du capteur (figure 4 : 50) le long de l'axe X, entre une position de repos, ou initiale, et une position de fin de course. Les signaux de commande peuvent également transiter par le câble *Ca,* par exemple sous forme de signaux binaires séries. Dans ce cas il est nécessaire que le bâti 6 comprennent des circuits (figure 4 : 56) aptes à reconnaître les signaux transmis par le micro-ordinateur 8 et les convertir en signaux de commande utilisables par les différents composants du capteur : signaux numériques et/ou analogiques. Le micro-ordinateur 8 reçoit également, via le câble *Ca,* des données sur la position du capteur le long de l'axe, et plus généralement sur l'état de fonctionnement des différents organes actifs présents dans le bâti 6.

Enfin, le micro-ordinateur 8 peut commander, par un dispositif d'interface spécialisé ou standard, une machine à commande numérique *MCN* ou une machine similaire pour la réalisation de chaussures ou de semelles orthopédiques, ou de façon plus générale pour reproduire l'objet tridimensionnel dont le profil d'au moins une partie de surface a été acquis. Le micro-ordinateur 8 peut être relié à cette machine *MCN* par un simple câble ou via un réseau de transmission.

Il doit être clair que les données représentant les coordonnées acquises peuvent être modifiées par un programme spécifique ou manuellement par un opérateur (à l'aide du clavier 82 par exemple), avant transmission ou enregistrement sur une disquette *DK.* En d'autres termes, l'objet n'est pas obligatoirement une reproduction du profil relevé mais dérivé de ce profil. Il peut, en effet, être souhaitable d'introduire des corrections, évolutives dans le temps, notamment pour des semelles orthopédiques, pour obtenir un redressement des malformations constatées. Dans ce dernier cas, l'historique des corrections apportées et des résultats obtenus sur un patient présentant des malformations peut être conservé en mémoire.

On va maintenant décrire deux modes de réalisation supplémentaires du dispositif d'acquisition de forme et de numérisation coopérant avec la boîte à aiguilles 1. Celle-ci reste inchangée et on peut mettre en oeuvre indifféremment l'une ou l'autre des variantes décrites en regard des figures 1A à 3.

Selon un premier mode de réalisation supplémentaire, décrit par référence aux figures 6A à 6C, on met en oeuvre un dispositif d'imagerie réalisé à base d'un appareil photographique numérique ou d'une caméra vidéo numérique. Cet appareil est référencé *CN* sur la figure 6A.

Pour fixer les idées, on considère que le dispositif palpeur 1 est conforme à la première variante, c'est-à-dire celle décrite en regard des figures 1A à 2, sans que cela soit limitatif. Il est donc inutile de le redécrire. On a également supposé que les aiguilles ou tiges 3 étaient à l'état de repos. Leurs extrémités inférieures 301 définissent une surface de référence comme précédemment, avantageusement un plan de référence *P*_{*R*}*.*

Ces extrémités 301 d'aiguilles 3 sont éclairées par au moins une source de lumière blanche, avantageusement par deux sources symétriques *L*₁ et *L*₂, sous incidence oblique. Quand elles sont éclairées, les faces terminales des aiguilles 3 apparaissent brillantes. Elles sont réfléchissantes à cet effet. Dans leur position au repos, l'appareil photographique *CN* observe donc une pluralité de points brillants de même intensité régulièrement répartis comme les aiguilles, et la densité lumineuse est sensiblement homogène sur toute la surface de référence *PR* si cette surface de référence est un plan. L'angle d'incidence moyen des rayons lumineux émis par les sources de lumière, *L*₁ et *L*₂, par rapport au plan de référence *PR,* est typiquement compris dans la gamme 30 à 60 degrés.

L'appareil *CN* convertit la brillance de chaque point en une série de signaux électriques de sortie de type numérique. Ces signaux sont transmis par une liaison 830 à une carte d'acquisition numérique 83, disposée à l'intérieur d'un appareil de traitement de signal à programme enregistré, avantageusement un micro-ordinateur similaire au micro-ordinateur 8 de la figure 5. La liaison 830 peut être par exemple du type série.

L'image photographiée par l'appareil *CN* peut être affichée sur l'écran 9 du micro-ordinateur 8, par exemple sous une forme "brute" comme le suggère la figure 6B. L'image 90a reproduite sur l'écran 9 se compose alors d'un ensemble de points affichés de même intensité lumineuse, symbolisés par des cercles blancs, sur la figure 6B. Cette image 90a représente la surface rectangulaire du plan de référence *P*_{*R*}*.* Chaque point affiché sur l'écran représente une des extrémités 301 des aiguilles 3.

Lorsqu'un pied est posé sur les extrémités supérieures 300 des aiguilles 3, comme illustré par la figure 1B, les aiguilles 3 s'enfoncent plus ou moins selon leur position dans la matrice. La densité lumineuse de la surface définie par les extrémités inférieures 301 des aiguilles 3 n'est plus homogène, et notamment la surface *S*_{*A*} délimitant la zone d'appui du pied 2. La densité varie de façon corrélative avec l'amplitude de l'enfoncement des aiguilles 3. En effet, l'angle d'incidence de la lumière émise par les sources, *L*₁ et *L*₂, sur les surfaces des extrémités des aiguilles va varier en fonction de l'amplitude de l'enfoncement, ce qui va modifier la réflexion de la lumière sur ces surfaces.

Les points composant l'image affichée 90b sur l'écran 9 ont désormais des intensités lumineuses variables, comme montré par la figure 6C. Pour illustrer le procédé de l'invention, on n'a représenté que deux valeurs d'intensité lumineuse, illustrées respectivement par des cercles blancs 900_{b} et noirs 901_{b}. Dans la réalité, on obtient une gradation beaucoup plus fine de valeurs d'intensité lumineuse, comprises entre une borne maximale (représentant par exemple l'état de repos : figure 1A) et une borne minimale (représentant un enfoncement maximum d'aiguille 3 : figure 1B).

Par un logiciel classique de traitement d'image du commerce, on peut déterminer en outre le contour de la surface *S*_{*A*} caractéristique de la surface d'appui du pied 2. Différentes méthodes sont connues. On peut pour ce faire calculer les gradients d'intensité lumineuse d'un point à un autre, et à partir de ces calculs déterminer le contour précité. En effet, en dehors de la surface *S*_{*A*}, les fluctuations d'intensité lumineuse sont nulles, ou pour le moins inférieures à un seuil déterminé. L'amplitude de l'intensité lumineuse est dérivée directement des signaux numériques en sortie de l'appareil *CN.*

De même, à l'intérieur de ce contour *S*_{*A*}, le logiciel précité peut déterminer l'état d'enfoncement en effectuant une corrélation entre l'intensité lumineuse acquise pour une aiguille donnée 3 et l'amplitude de son enfoncement. Le coefficient de corrélation peut être déterminé par un calibrage initial. Une première référence est obtenue au repos (plan de référence PR éclairé). Une ou plusieurs autres références peu(ven)t être obtenue(s) en enfonçant de façon connue et homogène l'ensemble des aiguilles 3, par exemple à l'aide d'un objet plan.

A partir de ces différentes déterminations, il est simple de construire un modèle en trois dimensions de la surface *S*_{*A*}*,* de l'isoler des autres points acquis (zone en dehors de la surface *S*_{*A*}*)* et d'afficher ce modèle sous forme "filaire" ou "lissée", comme précédemment. Ces opérations peuvent être réalisée sous la conduite du logiciel.

Enfin les données acquises peuvent être utilisées en temps réel et/ou enregistrées pour un usage ultérieur, ou encore être transmises à distance. Ces étapes du procédé ne diffèrent en rien de celles décrites en regard de la figure 5.

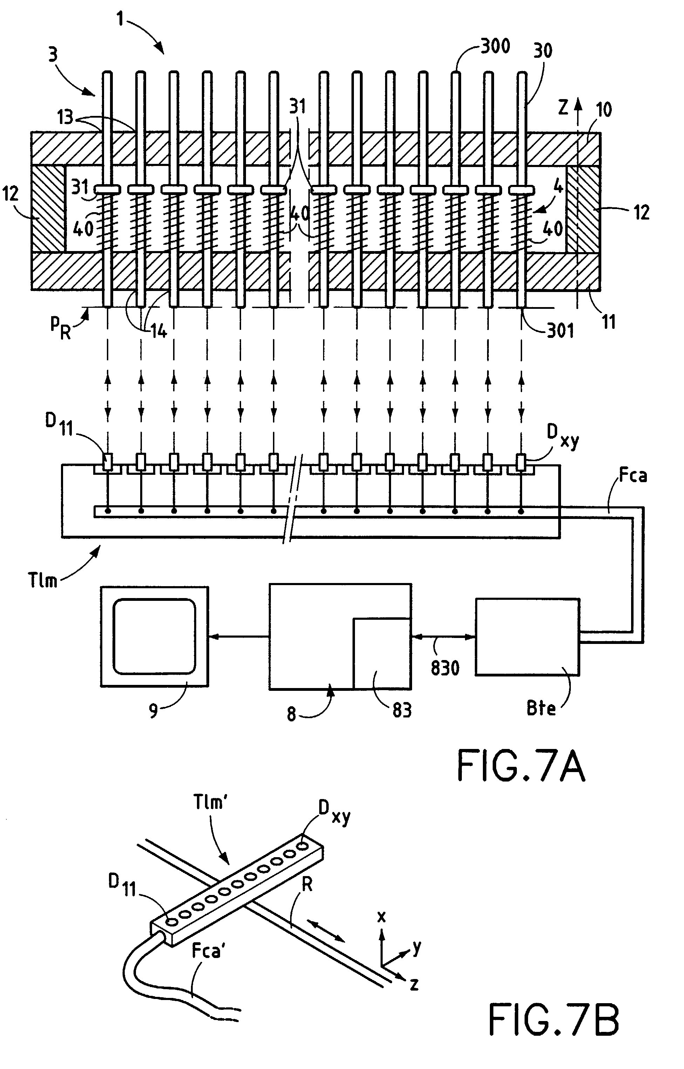

Selon un deuxième mode de réalisation supplémentaire, décrit par référence aux figures 7A et 7B, on met en oeuvre un dispositif de télémétrie. Ce mode de réalisation est lui-même susceptible de plusieurs variantes.

Dans la première variante de ce deuxième mode supplémentaire de réalisation, illustrée par la figure 7A, le télémètre *Tlm* comprend une matrice d'éléments optoélectroniques comprenant des diodes lasers semiconductrices, chacune étant associée à un élément photoconducteur. Les paires d'éléments opto-électroniques sont référencées *D*₁₁ à *D*_{*xy*}.

Comme précédemment, on a considéré que le dispositif palpeur 1 était du type représenté sur les figures 1A à 2, sans que cela soit limitatif. En outre, on a représenté ce dispositif 1 à l'état de repos, les extrémités inférieures 301 des aiguilles 3 définissant dans cet état un plan de référence *P*_{*R*}*.*

Le dispositif télémètre est disposé sous la boîte à aiguilles 1. Les diodes lasers des paires *D*₁₁ à *D*_{*xy*} de la matrice sont en nombre égal au nombre d'aiguilles 3, *x* et *y* étant égaux au nombre de rangées et de colonnes de la matrice d'aiguilles 3. Les diodes lasers des paires *D*₁₁ à *D*_{*xy*} sont arrangées spatialement de la même manière que les aiguilles 3. Le nombre total de diodes est donc égal au produit *xy*. Une diode laser particulière, par exemple la diode de la paire *D*_{*mn*} (*m* et *n* étant des indices arbitraires, inférieurs ou égaux à *x* et *y*, respectivement), émet un faisceau collimaté suivant l'axe *Z*, dirigé vers l'extrémité intérieure 301 de l'aiguille 3 placée en vis-à-vis, faisceau qui se réfléchit sur la face terminale de l'aiguille 3. La position des diodes lasers des paires *D*₁₁ à *D*_{*xy*} étant fixe dans l'espace, il est aisé de déterminer la distance séparant l'extrémité 301 d'une aiguille particulière 3 de la diode laser de la paire *D*_{*mn*} qui l'éclaire. L'élément photoconducteur associé à chaque diode laser de la paire *D*_{*mn*} convertit la lumière réfléchie en signal électrique transmis par un faisceau de câbles *fca* à des circuits électroniques *Bte,* représentés schématiquement sous la forme d'un boîtier. Ces circuits électroniques *Bte* commandent un balayage électronique des différentes diodes lasers des paires *D*₁₁ à *D*_{*xy*}, par exemple un balayage du type télévision. Les signaux de sortie délivrés par la matrice sont convertis en signaux numériques par ces mêmes circuits *Bte* et transmis, par une liaison de sortie 830, à une carte d'acquisition numérique 83, similaire à la carte de même référence de la figure 6A. Cette carte est disposée (dans l'exemple décrit) dans un micro-ordinateur 8, muni notamment d'un écran 9.

Lorsque le pied 2 est posé sur les extrémités supérieures 300 des aiguilles 3 (voir figure 1B), les aiguilles 3 s'enfoncent plus ou moins selon le processus déjà décrit. Le télémètre *Tlm* mesure directement les mouvements d'aiguilles 3, c'est-à-dire les variations de distance entre les extrémités inférieures 301 des aiguilles 3 et les diodes lasers correspondantes des paires *D*_{*mn*}. Les signaux de sortie et ceux transmis sur la liaison 830 sont directement représentatifs des amplitudes d'enfoncement. La gestion du balayage peut être commandée par le micro-ordinateur 8.

Après un calibrage initial, on peut donc afficher sur l'écran 9, comme précédemment, la surface *S*_{*A*} en trois dimensions. Cet affichage s'effectue à l'aide d'un logiciel classique de traitement d'image. Comme précédemment, les signaux de mesure acquis peuvent être utilisés immédiatement et/ou être enregistrés, ou encore être transmis à distance.

Selon une deuxième variante de ce mode supplémentaire de réalisation, illustrée par la figure 7B, le télémètre, référencé *Tlm',* comprend une simple barrette de paires de diodes lasers et d'élements photo-conducteurs, *D*₁₁ à *D*₁_{*y*}, disposée parallèlement à l'un des axes de la matrice d'aiguilles 3, par exemple l'axe *Y*. Le nombre de diodes lasers y est égal au nombre de rangées de la matrice d'aiguilles 3. Cette barrette de diodes lasers et d'éléments photo-conducteurs, *D*₁₁ à *D*₁_{*y*}, est entraînée par des moyens de motorisation (non représentés) semblables à ceux de la figure 4, suivant l'axe *X*. Le mouvement de translation est guidé par un ou plusieurs rails *R,* de manière à ce que la barrette reste parallèle à l'axe *Y*. Les moyens de motorisation peuvent comprendre avantageusement un moteur pas à pas. A chaque "pas" du mouvement de translation, on acquière une colonne de points, c'est-à-dire les distances séparant les diodes des extrémités inférieures 301 de la colonne d'aiguilles 3 en vis-à-vis.

Lorsque toutes les colonnes de la matrice d'aiguilles 3 ont été balayées, le processus d'acquisition est terminé. Le balayage mécanique suivant l'axe *X* peut être effectué dans n'importe quel sens. Les signaux de sortie du télémètre *Tlm',* à chaque pas d'acquisition, c'est-à-dire pour toutes les aiguilles d'une rangée, sont transmis par un faisceau de câble *fca'* à un boîtier électronique (non représenté), comme précédemment. Les processus de numérisation et d'utilisation des signaux de mesure acquis sont tout à fait similaires à ce qui a été précédemment décrit. La gestion des balayages électronique et mécanique peut être réalisée sous la conduite du micro-ordinateur 8.

Dans une autre variante encore, non représentée, la barrette de diodes et d'éléments photo-conducteurs, *D*₁₁ à *D*₁_{*y*}, est fixe. On effectue une déflexion des faisceaux lasers de manière à obtenir un balayage des extrémités inférieures 301 des aiguilles 3.

Plusieurs méthodes de télémétrie classiques peuvent être utilisées. Selon l'invention, et dans les trois variantes de réalisation qui viennent d'être décrites, la télémétrie est avantageusement basée sur l'émission d'un train d'impulsions lumineuses et des mesures de temps aller et retour, ce qui permet de déterminer des distances connaissant la vitesse de propagation de la lumière.

Enfin le télémètre optique peut être remplacé par un télémètre magnétique. Il est alors nécessaire que les aiguilles 3 soient en un matériau à propriété magnétique (acier par exemple).

A la lecture de ce qui précède, on constate aisément que l'invention atteint bien les buts qu'elle s'est fixés.

Il doit être clair cependant que l'invention n'est pas limitée aux seuls exemples de réalisation explicitement décrits, notamment en relation avec les figures 1 à 7.

En particulier, en lieu et place des ressorts hélicoïdaux 40, il est possible d'utiliser des organes similaires de rappel à propriété élastique exerçant à l'encontre de l'enfoncement des aiguilles une force de résistance qui est réglable, ce qui apporte un moyen de tarage variable en fonction du poids du patient.

Bien que des tiges de section circulaire soient particulièrement adaptées pour la réalisation de la boîte à aiguilles, car dans ce cas les orifices de passage à travers les plaques fermant la boîte, sont simples à réaliser, il n'en reste pas moins vrai que d'autres formes sont tout à fait concevables. On peut notamment prévoir des tiges de section carrée, rectangulaire ou hexagonale.

Les matériaux utilisables sont essentiellement liés à l'application spécifique envisagée et participent d'un simple choix technologique à la portée de l'Homme du Métier.

De même les exemples numériques n'ont été précisés que pour mieux illustrer l'invention.

Il doit être clair aussi que, bien que particulièrement adaptée à la réalisation de chaussures ou de semelles orthopédiques, on ne saurait cantonner l'invention à ce seul type d'applications. Elle s'applique au relevé d'au moins une portion de surface de tout objet tridimensionnel, pour peu qu'il puisse être appuyé, du fait de son propre poids ou par des moyens extérieurs, sur la face que l'on a appelée supérieure du dispositif palpeur, et que la portion de surface précitée ne dépasse pas les dimensions du cadre délimitant la matrice d'aiguilles. On a vu également que le dispositif palpeur pouvait être adapté à différents poids d'objets (ou de force d'appui) en modifiant le tarage des ressorts ou des organes qui en tiennent lieu. Ainsi, le système selon l'invention peut être utilisé dans toute technique demandant de réaliser le moule d'une pièce.

## Revendications

1. Système (7) de relevé d'une forme d'un objet tridimensionnel (2), notamment du profil d'une voûte plantaire (20), caractérisé en ce qu'il comprend un dispositif palpeur (1) comportant des tiges montées mobiles (3) dans un support (10, 11), sur une première extrémité (300) desquelles ledit objet (2) est susceptible d'être appliqué avec une force d'appui déterminée, de manière à déplacer lesdites tiges (3) en translation de sorte que l'ensemble des secondes extrémités (301) de ces tiges (3) définissent une surface *(S*_{*A*}*)* répliquant ladite forme à relever (20), des moyens élastiques (4) associés auxdites tiges mobiles (3) pour opposer une force de rappel s'opposant à ladite force d'appui, et un dispositif optique d'acquisition sans contact (5) de ladite surface (*S*_{*A*}), ledit dispositif (5) délivrant des signaux électriques de sortie (*V*_{*S*}) corrélés aux coordonnées dans l'espace des secondes extrémités (301) des tiges (3), par rapport à des coordonnées de référence *(P*_{*R*}*)* définies par la position prise par les tiges dans un état dit de repos.

2. Système selon la revendication 1, caractérisé en ce que ledit support du dispositif palpeur (1) est constitué d'une boîte à aiguilles (3) comportant des première (10) et seconde (11) parois principales planes, parallèles entre elles et munies d'orifices (13, 14) dans lesquels coulissent les corps (30) desdites aiguilles (3), de manière à ce qu'elles y soient guidées et qu'elles en dépassent de part et d'autre de ladite boîte, et des parois latérales ou entretoises (12) maintenant fixes lesdites première (10) et seconde (11) parois principales.

3. Système selon la revendication 1 ou 2, caractérisé en ce que ledit support du dispositif palpeur (1) est constitué d'une boîte à aiguilles (3) comportant des première (10') et seconde (11) parois principales, en ce que la première paroi principale (10') est pleine et en matériau déformable sous l'effet d'une force de pression, cette première paroi étant en contact avec lesdites premières extrémités (300) desdites tiges (3), et en ce que la seconde paroi (11) est munie d'orifices de guidage (14) dans lesquels coulissent les corps (30) desdites aiguilles (3), de manière à ce qu'elles dépassent de ladite boîte du côté de cette seconde paroi (11) par leurs secondes extrémités (301), et des parois latérales ou entretoises (12) maintenant lesdites première (10) et seconde (11) parois principales.

4. Système selon l'une quelconque des revendications 1 à 3, caractérisé en ce que ladite force de rappel exercée par lesdits moyens élastiques est réglable, notamment entre des valeurs prédéterminées dépendant du poids de l'utilisateur.

5. Système selon l'une quelconque des revendications précédentes, caractérisé en ce que lesdits moyens élastiques (4) sont constitués par des ressorts tarés (40), réalisés sous la forme de ressorts hélicoïdaux disposés coaxialement aux tiges (3) et comprimés entre une plaque fixe (11) de guidage desdites tiges et des butées (31) prévues sur chacun des corps (30) desdites tiges (3).

6. Système selon la revendication 5, caractérisé en ce qu'il est prévu une gamme de dispositifs palpeurs distincts (1), se différenciant les uns des autres par la valeur de tarage des moyens élastiques (4), qui sont interchangeables pour un même dispositif d'acquisition en fonction notamment du poids des personnes dont il s'agit de relever le profil de voûte plantaire.

7. Système selon la revendication 5, caractérisé en ce que ledit dispositif palpeur (1) comporte une plaque d'appui desdites butées (31) pour modifier simultanément le tarage de l'ensemble des ressorts (40).

8. Système selon l'une quelconque des revendications 1 à 7, caractérisé en ce que ledit dispositif d'acquisition sans contact (5) comprend un capteur optique (50) comportant au moins une source laser (51) générant un faisceau lamellaire *(F*_{*1*}*)* formant un plan laser de mesure dirigé vers lesdites extrémités inférieures (301) des tiges (3), des moyens de commande (55) du balayage dudit faisceau lamellaire *(F*_{*1*}*)* sur ces extrémités inférieures (301), et au moins une caméra optoélectronique (52, 53) analysant la trace de ce faisceau *(F*_{*1*}*)* sur les extrémités inférieures balayées (301) et délivrant des signaux de sortie (*V*_{*S*}) représentant les coordonnées des secondes extrémités (301) de tiges (3) par rapport auxdites coordonnées de référence *(P*_{*R*}*).*

9. Système selon l'une quelconque des revendications 1 à 7, caractérisé en ce que ledit dispositif d'acquisition comprend au moins une source de lumière (*L*₁, *L*₂) éclairant lesdites extrémités inférieures (301) des tiges (3) et un appareil photographique (*CN*) ou une caméra délivrant des signaux de sortie numériques représentant les variations d'intensité de la lumière réfléchie par les faces terminales inférieures des tiges (3), ainsi que des circuits électroniques (8, 83) déterminant le profil à relever à partir desdites variations d'intensité lumineuse par rapport à l'intensité lumineuse au repos.

10. Système selon l'une quelconque des revendications 1 à 7, caractérisé en ce que ledit dispositif d'acquisition comprend des moyens optiques de télémétrie *(Tlm)* disposés en regard desdites extrémités inférieures (301) des tiges (3), mesurant la distance les séparant des extrémités inférieures (301) des tiges (3) et délivrant des signaux électriques de sortie représentant ces distances.

11. Système selon l'une quelconque des revendications 1 à 10, caractérisé en ce que lesdites tiges (3) sont arrangées selon les lignes et les colonnes d'une matrice à maille rectangulaire.

12. Système selon la revendication 10, caractérisé en ce que, lesdites tiges (3) étant arrangées selon les lignes et les colonnes d'une matrice rectangulaire, lesdits moyens de télémétrie *(Tlm, Tlm')* comprennent des éléments optoélectroniques à diodes lasers semiconductrices (*D*₁₁-*D*_{*xy*}), groupés sur une barrette entraînée dans un mouvement de balayage par rapport aux extrémités inférieures de tiges ou répartis suivant la même matrice que lesdites tiges, ainsi que des organes photoconducteurs respectivement associés auxdites diodes pour délivrer des signaux de sortie représentatifs des distances détectées par lesdites diodes lasers (*D*₁₁-*D*_{*xy*}) au moyen d'un faisceau émis en direction d'une extrémité inférieure (301) de tige (3) correspondante.

13. Procédé de réalisation d'une chaussure ou d'une semelle orthopédique pour un pied déterminé, le procédé mettant en oeuvre le système de relevé d'une forme d'un objet tridimensionnel (2) selon l'une quelconque des revendications précédentes, caractérisé en ce que, lesdites tiges (3) étant arrangées selon une matrice rectangulaire de *M* lignes et *N* colonnes, il comprend au moins les étapes suivantes :
a/ pose dudit pied (2) sur ledit dispositif palpeur (1), la voûte plantaire (20) en appui, total ou partiel, sur les premières extrémités des tiges (3), dites supérieures (300), dans une posture déterminée, de manière à exercer ladite force d'appui ;
b/ acquisition d'un nuage de points correspondant à *M* tranches de *N* points chacune, numérisation et génération desdits signaux de sortie (*V*_{*S*}) pour ces points acquis par conversion opto-électronique, de manière à relever les coordonnées desdits points acquis dans un espace de mesure déterminé ;
c/ et transformation desdites données acquises et numérisées dans un référentiel absolu par comparaison avec des données de coordonnées de référence appartenant audit espace de mesure, de manière à en déduire le profil de ladite voûte plantaire (20) ;
en ce qu'il comprend en outre une étape préliminaire de calibrage dudit dispositif d'acquisition (5, *Tlm, Tlm')* consistant à relever les coordonnées desdites extrémités inférieures (301) des tiges (3) lorsqu'elles sont dans ledit état de repos pour lequel lesdites extrémités supérieures (300) ne sont pas soumises à ladite force d'appui, l'ensemble des extrémités inférieures (301) définissant une surface de référence (*P*_{*R*}) dans ledit espace de mesure ;
et en ce que, par action préalable sur le tarage de l'ensemble de ressorts (40) associés aux différentes tiges pour constituer lesdits moyens élastiques (4), on règle la force de rappel s'exerçant à l'encontre du déplacement desdites tiges sous l'effet de ladite force d'appui, en fonction du poids du patient.
